# FASCICULE DE BREVET EUROPEEN

(11) **EP 3 802 472 B1**
(45) Date de publication et mention de la délivrance du brevet: **11.12.2024**
(21) Numéro de dépôt: 19725172.1
(22) Date de dépôt: 21.05.2019
(51) Int. Cl.: C07C 29/74, C07C 29/80, C07C 29/86, C07C 31/08, C07C 31/12, B01D 11/04, B01D 3/14

(54) **MÉTHODE D'EXTRACTION PARTIELLE DE BUTANOL D'UNE SOLUTION AQUEUSE COMPRENANT DE L'ÉTHANOL ET DU BUTANOL**
TEILEXTRAKTION VON BUTANOL AUS EINER WÄSSRIGEN LÖSUNG MIT ETHANOL UND BUTANOL
PARTIAL EXTRACTION OF BUTANOL FROM AN AQUEOUS SOLUTION CONTAINING ETHANOL AND BUTANOL

(30) Priorité: 01.06.2018 FR 1854791
(43) Date de publication de la demande: 14.04.2021
(73) Titulaire: IFP Energies nouvelles, 92852 Rueil-Malmaison (FR); COMPAGNIE GENERALE DES ETABLISSEMENTS MICHELIN, 63000 Clermont-Ferrand (FR)
(72) Inventeur: FISCHER, Beatrice, 92852 RUEIL-MALMAISON (FR); DASTILLUNG, Rejane, 92852 RUEIL-MALMAISON (FR)
(74) Mandataire: IFP Energies nouvelles
(86) Numéro de dépôt international: PCT/EP2019/063137
(87) Numéro de publication internationale: WO 2019/228871

(56) Documents cités:
- WO-A1-2016/054706
- FR-A1- 2 549 043
- FR-A1- 3 026 100

## Description

### DOMAINE TECHNIQUE DE L'INVENTION

L'invention concerne un procédé de traitement d'une charge hydroalcoolique comprenant de l'éthanol et du butanol, le butanol étant de préférence en faible quantité par rapport à l'eau et à l'éthanol, permettant d'extraire partiellement le butanol et de produire un effluent riche en eau, un effluent riche en éthanol et un effluent riche en butanol. Ce procédé apparait particulièrement intéressant pour le traitement de l'effluent hydroalcoolique du procédé Lebedev.

### ETAT DE LA TECHNIQUE

Le traitement de solutions aqueuses comprenant de l'éthanol est connu pour être difficile. La séparation de l'eau et de l'éthanol par distillation est en effet gênée par la formation d'un azéotrope eau-éthanol qui limite la récupération d'éthanol pur en tête de colonne. La présence de butanol dans le mélange eau-éthanol, même en petites quantités, complique encore la séparation par distillation puisqu'il y a formation d'un deuxième azéotrope, l'azéotrope eau-butanol. Lorsque le mélange contient en outre d'autres impuretés, comme l'effluent eau-éthanol du procédé Lebedev, les problèmes de séparation sont aggravés.

Il apparait donc intéressant d'extraire le butanol, au moins partiellement, afin de produire à partir de solutions hydroalcooliques comprenant de l'eau, de l'éthanol et du butanol, en particulier lorsque ce dernier est en faible quantité, un effluent riche en éthanol et un effluent riche en eau.

Le document FR 2 549 043 décrit un procédé de fractionnement d'une solution aqueuse comprenant du butanol et de l'acétone ou un mélange acétone-éthanol, pour produire un effluent comprenant essentiellement de l'eau, un effluent butanol pratiquement pur et un effluent riche en acétone ou mélange acétone-éthanol. Le procédé comprend une section de distillation alimentée par la solution aqueuse de butanol et d'acétone à laquelle a été mélangée une phase eau-butanol recyclée, et une deuxième section de distillation, alimentée par le distillat de tête de la première et comprenant un système de séparation, notamment par décantation, qui permet le soutirage et le recyclage d'une phase eau-butanol.

Cependant, la solution aqueuse traitée par le procédé décrit dans le document FR 2 549 043 est relativement pauvre en acétone, ou en mélange acétone-éthanol, et ne donne aucun renseignement sur une possible transposition au traitement d'autres mélanges aqueux ternaires comprenant du butanol, en particulier des mélanges hydroalcooliques comprenant de l'eau, de l'éthanol et du butanol comprenant de faibles quantités de butanol.

La présente invention propose un procédé pour traiter des mélanges eau-éthanol-butanol, notamment comprenant de faibles quantités de butanol, permettant de produire un effluent riche en eau, un effluent riche en éthanol et un effluent butanol pratiquement pur.

Ce procédé apparait particulièrement intéressant pour le traitement de l'effluent hydroalcoolique du procédé Lebedev puisque l'éthanol séparé sera plus concentré. Il pourra alors être recyclé et servir à nouveau de réactif.

En effet, dans le procédé Lebedev qui permet de produire du butadiène par conversion de l'éthanol, comme décrit par exemple dans le livre « Synthetic rubber », chapitre 4, W.J. Toussaint et J. Lee Marah, ou plus récemment dans les documents FR 3 026 100 et FR 3 026 101, le taux de conversion en butadiène par passe est limité. Le recyclage des réactifs non convertis, en particulier l'éthanol, apparait donc nécessaire notamment pour des raisons économiques. L'éthanol doit donc être séparé autant que possible de l'eau produite (2 moles d'eau environ pour une mole de butadiène), avant d'être recyclé vers le réacteur. L'eau limite en effet la réactivité et la sélectivité du catalyseur réactionnel. Elle induit également un grossissement des équipements et une augmentation de la consommation des utilités.

De plus, de nombreuses impuretés, dont du butanol, sont générées lors de la déshydratation. Or le butanol, difficile est à éliminer, sauf à consommer beaucoup d'énergie et de polluer l'eau soutirée lors du traitement des effluents dans le procédé Lebedev. Il est donc recyclé avec l'éthanol et renvoyé dans le réacteur. Le butanol réagit très partiellement dans le réacteur et s'accumule au fur et à mesure des recyclages jusqu'à une quantité de butanol transformé égale à la quantité de butanol formé à partir d'éthanol. La transformation du butanol induit, de fait, une baisse de la sélectivité du catalyseur réactionnel du procédé Lebedev et donc une baisse de rendement global en butadiène. Par ailleurs, le butanol recyclé entraine avec lui une quantité d'eau non négligeable, ce qui implique une augmentation de la taille des équipements et de la consommation des utilités et des difficultés de séparation des réactifs éthanol et acétaldéhyde.

L'objet de la présente invention est de pallier à ces inconvénients, en fournissant un procédé de traitement de solutions hydroalcooliques eau-éthanol comprenant du butanol, notamment en faible quantité. En particulier, la présente invention propose une méthode de traitement d'une charge hydroalcoolique comprenant, outre l'eau et l'éthanol, du butanol, de préférence en faible quantité par rapport à l'eau et l'éthanol, par extraction partielle du butanol, de manière à produire un effluent riche en éthanol et un effluent riche en eau, aptes à être recyclés, sans perte significative d'éthanol et sans engendrer les problèmes évoqués cidessus.

### RESUME DE L'INVENTION

La présente invention concerne un procédé de traitement d'une charge hydroalcoolique comprenant de l'eau, de l'éthanol et du butanol, pour produire un effluent riche en éthanol, un effluent riche en eau et un effluent riche en butanol, comprenant :
a) une étape de séparation eau-éthanol, comprenant une section de séparation eau-éthanol comprenant une colonne de distillation comprenant au moins 14 plateaux théoriques et ayant un taux de reflux molaire inférieur ou égal à 1,2, ladite section de séparation eau-éthanol comprenant :
   ∘ une alimentation en ladite charge hydroalcoolique, située au niveau ou en aval du troisième plateau théorique de la colonne ,
   ∘ le soutirage latéral d'une solution hydroalcoolique comprenant du butanol, dans la zone d'accumulation du butanol de la colonne de distillation, située en aval de l'alimentation,
   ∘ une injection de la phase lourde issue de l'étape b), située en aval et proche du soutirage,
   ∘ une injection du distillat éthanol-eau issu de l'étape c), située en amont de l'alimentation et proche de la tête de ladite colonne de distillation,
   ladite section de distillation produisant en tête de colonne un effluent riche en éthanol et en fond de colonne un effluent riche en eau ;
b) une étape de démixtion comprenant
   ∘ une section de mélange de ladite solution hydroalcoolique soutirée à l'étape a) avec la coupe eau/butanol/éthanol soutirée à l'étape c) ;
   ∘ une section de décantation du mélange précédent comprenant un ballon de décantation dans lequel la pression est inférieure ou égale à 6 bars absolus, ladite section de décantation produisant une phase lourde comprenant principalement de l'eau et une phase légère ;
c) une étape de séparation du butanol, comprenant une section de distillation du butanol comprenant au moins une colonne de distillation alimentée par la phase légère issue de l'étape b) et comprenant un soutirage latéral, situé sur ladite colonne dans une zone comprise entre l'alimentation et le fond de la colonne, d'une coupe eau/butanol/éthanol, ladite section de distillation du butanol produisant en fond de colonne un effluent riche en butanol et en tête de colonne un distillat éthanol-eau.

Il a ainsi été découvert, de manière surprenante, qu'il est possible de créer, dans certaines conditions, une accumulation de butanol dans la colonne de distillation eau-éthanol. Les inventeurs ont aussi mis en évidence qu'un soutirage au niveau de cette zone combiné à un système élaboré de séparations et de recyclages permet de produire un effluent enrichi en éthanol et un effluent riche en eau qui pourront être recyclés. Par exemple, dans le cas du procédé Lebedev, l'effluent riche en éthanol pourra être recyclé comme réactif vers la section réactionnelle du procédé Lebedev et l'eau vers une section de lavage, limitant ainsi la consommation d'utilités et la taille des équipements.

Avantageusement, le procédé selon l'invention permet de produire un effluent aussi concentré en éthanol que possible, sans perte d'éthanol, tout en limitant la consommation d'énergie.

Le procédé selon l'invention permet également de récupérer un effluent butanol pratiquement pur et donc valorisable et un effluent d'eau exempt de butanol.

L'invention s'applique avantageusement au traitement de l'effluent eau-éthanol du procédé Lebedev. L'effluent eau-éthanol issu de l'unité réactionnelle du procédé Lebedev comprend outre l'eau et l'éthanol, du butanol, sous-produit de la réaction de conversion de l'éthanol en butadiène, en faible quantité. La distillation eau-éthanol, dans un procédé Lebedev, peut ainsi être optimisée par extraction partielle du butanol selon le procédé de l'invention : l'effluent riche en éthanol extrait en tête est ainsi plus concentré en éthanol et peut être recyclé vers le réacteur ; la quantité de butanol recyclée diminue par rapport à un procédé Lebedev dit classique dans lequel le butanol n'est pas extrait. Ainsi, l'accumulation du butanol dans le réacteur de production du butadiène ne se produit donc plus, ou moins rapidement. La quantité d'eau recyclée avec l'effluent riche éthanol est elle-aussi réduite. Le coût énergétique du procédé Lebedev global, intégrant le procédé selon l'invention, est donc limité.

### DESCRIPTION DE L'INVENTION

Selon l'invention, la numérotation des plateaux théoriques et le positionnement des différents éléments, notamment dans la section de distillation eau-éthanol, avec les termes tels que en « amont » / « aval » s'effectuent dans le sens d'écoulement du liquide dans la colonne de distillation, c'est-à-dire de la tête vers le fond de la colonne.

Par procédé Lebedev, on entend un procédé de conversion de l'éthanol en butadiène, comme celui décrit par exemple dans le document documents FR 3 026 100 ou FR 3 026 101.

Selon la présente invention, l'expression « compris entre ... et ... » signifie que les valeurs limites de l'intervalle sont incluses dans la gamme de valeurs décrite. Si tel n'était pas le cas et que les valeurs limites n'étaient pas incluses dans la gamme décrite, une telle précision serait apportée par la présente invention.

La présente invention consiste en un procédé de traitement d'une charge hydroalcoolique comprenant de l'eau, de l'éthanol et du butanol, pour produire un effluent riche en éthanol, un effluent riche en eau et un effluent riche en butanol, comprenant :
a) une étape de séparation eau-éthanol, comprenant une section de séparation eau-éthanol comprenant une colonne de distillation comprenant au moins 14 plateaux théoriques et ayant un taux de reflux molaire inférieur ou égal à 1,2 , ladite section de séparation eau-éthanol comprenant :
   ∘ une alimentation en ladite charge hydroalcoolique, située au niveau ou en aval du troisième plateau théorique de la colonne,
   ∘ le soutirage latéral d'une solution hydroalcoolique comprenant du butanol, dans la zone d'accumulation du butanol de la colonne de distillation, située sur la colonne de distillation en aval de l'alimentation,
   ∘ une injection de la phase lourde issue de l'étape b), située en aval et proche du soutirage,
   ∘ une injection du distillat éthanol-eau issu de l'étape c), située en amont de l'alimentation et proche de la tête de ladite colonne de distillation,
   ladite section de distillation produisant en tête de colonne un effluent riche en éthanol et en fond de colonne un effluent riche en eau ;
b) une étape de démixtion comprenant
   ∘ une section de mélange de ladite solution hydroalcoolique soutirée à l'étape a) avec la coupe eau/butanol/éthanol soutirée à l'étape c) ;
   ∘ une section de décantation du mélange précédent comprenant un ballon de décantation dans lequel la pression est inférieure ou égale à 6 bars absolus, ladite section de décantation produisant une phase lourde comprenant principalement de l'eau et une phase légère ;
c) une étape de séparation du butanol, comprenant une section de distillation du butanol comprenant au moins, une colonne de distillation alimentée par la phase légère issue de l'étape b) et comprenant un soutirage latéral situé sur la colonne butanol dans une zone comprise entre l'alimentation et le fond de la colonne, d'une coupe eau/butanol/éthanol, ladite section de distillation produisant en fond de colonne un effluent riche en butanol et en tête de colonne un distillat éthanol-eau.

De préférence, le procédé selon l'invention consiste en a) l'étape de séparation eau-éthanol, b) l'étape de démixtion et c) l'étape de distillation du butanol, précédemment citées.

### La charge hydroalcoolique

Le procédé selon l'invention s'applique au traitement de charges hydroalcooliques comprenant de l'eau, de l'éthanol et du butanol, le butanol étant de préférence en faible quantité par rapport à l'eau et à l'éthanol. Le butanol représente notamment moins de 5% mol/mol, en particulier moins de 2% mol/mol, plus particulièrement moins de 1% mol/mol ou encore moins de 0,5% mol/mol de la charge hydroalcoolique à traiter.

La charge hydroalcoolique traitée par le procédé selon l'invention est une charge liquide, de préférence une solution ou suspension.

La charge hydroalcoolique, comprenant de l'eau, de l'éthanol et du butanol, peut également contenir d'autres impuretés, en général dans des proportions inférieures à celle du butanol. Les impuretés peuvent par exemple être des hydrocarbures, saturés ou insaturés, voire aromatiques ou des produits oxygénés, saturés ou insaturés, voire aromatiques, autres que le butanol.

Avantageusement, lorsque le procédé selon l'invention est intégré dans un procédé Lebedev, la charge hydroalcoolique est l'effluent eau-éthanol récupéré en sortie d'unité de conversion de l'éthanol en butadiène, après séparation du butadiène, lavage de l'effluent liquide et séparation de l'acétaldéhyde et d'autres impuretés légères.

### Etape a) de séparation eau-éthanol

Le procédé selon l'invention comprend une étape a) de séparation eau-éthanol, comprenant une section de séparation eau-éthanol. La section de séparation eau-éthanol comprend une colonne de distillation comprenant au moins 14 plateaux théoriques. Avantageusement, la colonne de distillation de la section de séparation eau-éthanol comprend entre 14 et 40 plateaux théoriques, de préférence entre 20 et 35 plateaux théoriques et plus préférentiellement encore entre 24 et 33 plateaux théoriques.

Avantageusement, la colonne de distillation de la section de séparation eau-éthanol comporte un dispositif de reflux (ou dispositif de condensation) en tête et un dispositif de rebouillage en fond. La colonne de distillation fonctionne à un taux de reflux molaire inférieur ou égal à 1,2, de préférence compris entre 0,25 et 1,2, de façon préférée compris entre 0,5 et 1,15 et plus préférentiellement encore compris entre 0,65 et 1,15.

La colonne de distillation de la section de séparation eau-éthanol est alimentée, de préférence en continu, en ladite charge hydroalcoolique en un point intermédiaire de la colonne de distillation situé au niveau ou en aval du troisième plateau théorique (ou plateau théorique numéro 3). De préférence, la colonne est alimentée en ladite charge hydroalcoolique dans une zone comprise entre le quart et les trois quarts de la hauteur totale de ladite colonne, de façon préférée dans une zone comprise entre le tiers et les deux tiers de la hauteur totale de ladite colonne.

La zone située entre l'alimentation et le fond de la colonne est appelée zone de fond.

Les inventeurs ont alors découvert que, dans ces conditions de fonctionnement de la colonne de distillation, et avec de telles caractéristiques d'architecture de la colonne, qui avantageusement restent économiquement raisonnables, une zone d'accumulation du butanol se formait, en particulier dans une zone située en aval de l'alimentation.

Conformément à l'invention, la section de séparation eau-éthanol comprend un soutirage latéral d'une solution hydroalcoolique comprenant du butanol. La concentration en butanol de la solution soutirée dépend de plusieurs paramètres tant au niveau de la composition de la charge que des caractéristiques de la colonnes de distillation. Cependant, la solution soutirée comprend de préférence au moins 2% mol/mol, au moins 4% mol/mol, au moins 7% mol/mol, au moins 10% mol/mol de butanol, et avantageusement moins de 60% mol/mol, en particulier moins de 50% mol/mol de butanol. Cette solution hydroalcoolique soutirée comprend également au moins de l'eau et éventuellement de l'éthanol.

Selon l'invention, le soutirage latéral se situe dans la zone d'accumulation du butanol de la colonne de distillation de la section de séparation eau-éthanol, en aval de l'alimentation, c'est-à-dire dans la zone de fond de la colonne de distillation telle que définie plus haut. Avantageusement, le soutirage latéral se situe dans une zone en aval de l'alimentation, comprise entre le quart et les trois quarts de la hauteur de la zone de fond, de préférence comprise entre le tiers et les deux tiers de la hauteur de la zone de fond. La position du soutirage latéral sur la colonne est définie, selon l'invention, dans le sens de l'écoulement du liquide dans ladite colonne de distillation eau-éthanol, c'est-à-dire de la tête vers le fond de la colonne.

Dans l'étape a) de séparation eau-éthanol du procédé selon l'invention, la section de séparation eau-éthanol comprend en outre avantageusement deux injections permettant de recycler l'eau et éventuellement l'éthanol soutirés. En particulier, la section de séparation eau-éthanol comprend :
- une injection de la phase lourde issue de l'étape b) de démixtion, située en aval et proche du soutirage latéral, de préférence dans une zone comprise entre le soutirage latéral et le troisième plateau théorique en aval dudit soutirage latéral, et
- une injection du distillat éthanol-eau issu de l'étape c), située en amont de l'alimentation et proche de la tête de la colonne de distillation, de préférence dans la zone de la colonne de distillation comprise entre le plateau théorique en tête de colonne au niveau duquel revient le reflux et le troisième plateau théorique.

Les dispositifs de reflux (ou condensation) en tête de colonne et de rebouillage en fond de colonne pouvant être utilisés dans l'étape a) de séparation eau-éthanol du procédé selon l'invention sont ceux bien connus de l'Homme du métier. Avantageusement, le dispositif de reflux en tête de colonne comprend un échangeur de chaleur utilisant de l'eau de refroidissement ou de l'air, un ballon de reflux et une pompe de reflux permettant de renvoyer le reflux à la colonne de distillation. Avantageusement, le dispositif de rebouillage utilise de la vapeur basse pression.

Selon l'invention, la section de distillation de l'étape a) produit en tête un effluent riche en éthanol et en fond un effluent riche en eau.

Avantageusement, l'effluent riche en éthanol extrait en tête de colonne eau-éthanol dans l'étape a) du procédé selon l'invention comprend au moins de l'éthanol à une teneur supérieure ou égale à 60% mol/mol, de préférence supérieure ou égale à 65% mol/mol et plus préférentiellement encore supérieure ou égale 70% mol/mol. L'effluent riche en éthanol peut comprendre au moins de l'eau. Dans le cas où le procédé selon l'invention est intégré dans un procédé Lebedev, l'effluent riche en éthanol peut être recyclé vers la section réactionnelle dans laquelle l'éthanol sera consommé comme réactif.

L'effluent riche en eau produit en fond de colonne contient essentiellement de l'eau, c'est-à-dire au moins 95% molaire d'eau, de préférence au moins 98% molaire d'eau, préférentiellement au moins 99% molaire d'eau. Avantageusement, l'effluent riche en eau contient moins de 10 ppm molaire, de préférence moins de 5 ppm molaire, plus préférentiellement moins de 1 ppm molaire et de façon encore plus préférée moins de 0,1 ppm molaire d'éthanol, et moins de 10 ppm molaire, de préférence moins de 5 ppm molaire, préférentiellement moins de 1 ppm molaire de butanol, et de manière préférée est exempt de butanol.

### Etape b) de démixtion

Le procédé selon l'invention comprend une étape b) de démixtion comprenant une section de mélange de la solution hydroalcoolique soutirée lors de l'étape a) avec une coupe eau/butanol/éthanol soutirée à l'étape c), et une section de décantation du mélange comprenant un ballon de décantation dont la pression est inférieure ou égale à 6 bars absolus. L'étape b) de démixtion produit une phase lourde comprenant principalement de l'eau et une phase légère.

Dans la section de mélange, la solution hydroalcoolique soutirée à l'étape a) dans la zone d'accumulation du butanol de la colonne de distillation est mélangée avec une coupe eau/butanol/éthanol soutirée à l'étape c). Avantageusement, la coupe eau/butanol/éthanol soutirée à l'étape c) contient peu d'éthanol et est concentrée en butanol. De préférence, la coupe eau/butanol/éthanol soutirée à l'étape c) comprend entre 15 et 45% mol/mol, de préférence entre 20 et 40 % mol/mol de butanol, par exemple entre 25 et 35% mol/mol de butanol, moins de 3% mol/mol d'éthanol, de préférence moins de 1% mol/mol d'éthanol, et de l'eau. Avantageusement, la solution hydroalcoolique soutirée à l'étape a) représente entre 15 et 40 % mol/mol du mélange de la section de mélange de l'étape b) du procédé selon l'invention et la coupe eau/butanol/éthanol soutiré à l'étape c) entre 60 et 85 % mol/mol du mélange de la section de mélange de l'étape b) du procédé selon l'invention.

La section de mélange permet d'obtenir un mélange de composition telle qu'elle se trouve dans la zone de séparation de phases, appelée aussi « loupe de démixtion », du diagramme ternaire eau/éthanol/butanol.

Avantageusement, la pression à l'intérieur du ballon de décantation est à une pression inférieure ou égale à 6 bars absolus. Plus particulièrement, la pression à l'intérieur du ballon de décantation est comprise entre 1 et 6 bars absolus, de préférence entre 1 et 4 bars absolus, plus préférentiellement entre 1 et 2 bars absolus et notamment égale à 1,5 bars absolus.

Dans la section de décantation, dans les conditions opératoires de l'invention, le mélange obtenu à l'issue de la section de mélange de l'étape b) du procédé selon l'invention va se séparer en deux phases :
- une phase aqueuse, la phase lourde, comprenant principalement de l'eau, c'est-à-dire de préférence au moins 85% molaire d'eau, plus particulièrement entre 85-99% molaire d'eau, notamment entre 90-98% molaire d'eau, et
- une phase légère avantageusement concentrée en butanol, comprenant en particulier moins de 15% mol/mol d'éthanol, entre 60 et 85% mol/mol d'eau et au moins 5% mol/mol de butanol, de préférence entre 5% et 40% mol/mol de butanol, préférentiellement entre 10% et 40% mol/mol de butanol et notamment entre 15% et 38% mol/mol de butanol.

La phase lourde est avantageusement recyclée et renvoyée vers la colonne de distillation eau-éthanol de l'étape a) dans laquelle elle est réinjectée. La phase légère quant à elle est récupérée et traitée à l'étape suivante, l'étape c), du procédé selon l'invention.

Dans un mode préférée de l'invention, le mélange préparé dans la section de mélange est refroidi, par exemple dans un échangeur de chaleur utilisant de l'eau de refroidissement ou de l'air, de préférence en dessous de son point de bulle, par exemple en dessous de 95°C, notamment entre 80 et 95°C, de préférence entre 85 et 95°C,avant d'être envoyé dans le ballon de décantation.

### Etape c) de distillation et séparation du butanol

Le procédé selon l'invention comprend une étape c) de séparation du butanol comprenant une section de distillation et produisant en tête de colonne un distillat éthanol-eau et en fond de colonne un effluent riche en butanol. La colonne de distillation de la section de distillation du butanol, appelée colonne butanol selon l'invention, est alimentée par la phase légère issue de l'étape b) en un point intermédiaire entre la tête et le fond. Cette colonne comprend également un soutirage latéral situé dans une zone entre l'alimentation et le fond de la colonne d'une coupe eau/butanol/éthanol.

L'Homme du métier saura facilement dimensionner la colonne de distillation utilisée dans cette étape de séparation du butanol. Classiquement, la colonne de séparation du butanol comprend entre 10 et 30 plateaux théoriques, a un reflux molaire compris entre 0,5 et 15 et est alimentée dans la zone située dans sa moitié supérieure.

Avantageusement, la coupe eau/butanol/éthanol soutirée comprend entre 15 et 45% mol/mol de butanol, de préférence 20 et 40 % mol/mol de butanol, par exemple entre 25 et 35% mol/mol de butanol, moins de 5% mol/mol d'éthanol, de préférence moins de 3% mol/mol d'éthanol, de façon plus préférée moins de 1% mol/mol d'éthanol, et de l'eau. Conformément à l'invention, le liquide soutiré est recyclé vers la section de mélange de l'étape b) du procédé selon l'invention.

Le distillat éthanol-eau extrait en tête de colonne à l'étape c) du procédé selon l'invention comprend essentiellement un mélange d'éthanol et d'eau, le mélange éthanol-eau représentant au moins 80% molaire, de préférence au moins 90% molaire et de façon encore plus préférée au moins 95% molaire du distillat extrait. Avantageusement, l'éthanol représente au moins 50% mol/mol dudit distillat éthanol-eau, de préférence au moins 60% mol/mol et de façon encore plus préférée au moins 70% mol/mol dudit distillat éthanol-eau. Ledit distillat éthanol-eau, extrait en tête de colonne de distillation du butanol, est avantageusement renvoyé vers la colonne de distillation eau-éthanol de l'étape a) du procédé selon l'invention, permettant ainsi de limiter les pertes en éthanol.

L'effluent extrait en fond de colonne dans la section de distillation du butanol de l'étape c) du procédé selon l'invention est riche en butanol. Il comprend notamment au moins 90% molaire, de préférence au moins 95% molaire et encore plus préférentiellement au moins 97% molaire de butanol. Cet effluent étant du butanol pratiquement pur pourra être valorisé.

La section de distillation du butanol peut comprendre, en outre, un dispositif de reflux en tête, comprenant par exemple un échangeur de chaleur utilisant de l'eau de refroidissement ou de l'air, un ballon de reflux et une pompe de reflux permettant de renvoyer le reflux à la colonne de distillation du butanol et le distillat liquide vers la colonne de séparation eau/éthanol de l'étape a) du procédé selon l'invention. La section de distillation du butanol peut comprendre également un dispositif de rebouillage en fond, utilisant par exemple de la vapeur basse pression.

Le procédé selon l'invention sera mieux compris au vu des exemples 1 à 4 suivants et des figures, qui sont donnés à titre illustratif et non limitatif.

### FIGURES

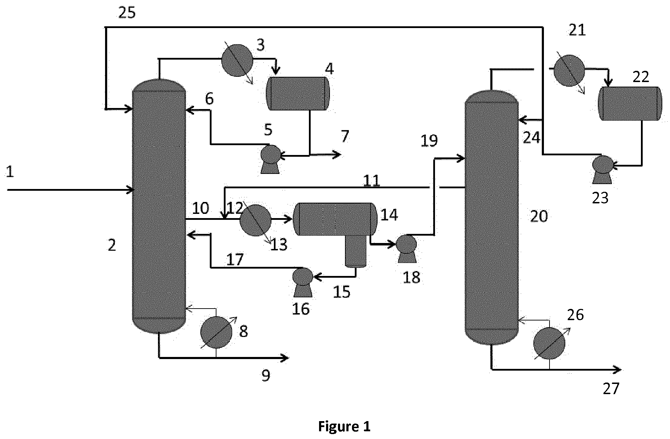

Figure 1 : Schéma d'un mode de réalisation du procédé selon l'invention.

### Description de la Figure 1 :

La charge hydroalcoolique alimente par le conduit (1) la colonne (2) de séparation eau-éthanol. La colonne (2) comprend un dispositif de reflux (ou condensation) en tête, comprenant par exemple un échangeur de chaleur (3) utilisant de l'eau de refroidissement ou de l'air, un ballon de reflux (4) , une pompe de reflux (5) renvoyant le reflux à la colonne (2) par le conduit (6), et un dispositif de rebouillage (8) en fond. L'effluent riche éthanol est extrait en tête de la colonne (2) par le conduit (7). L'effluent riche eau est extrait par le conduit (9) en fond de la colonne (2). La colonne (2) comprend également un soutirage latéral (10) installé dans la zone d'accumulation du butanol et deux injections de retour, l'une (25) proche de la tête et l'autre (17) en aval et proche du soutirage.

La solution hydroalcoolique soutirée et sortant de la colonne (2) de séparation par le conduit (10) est mélangée avec une coupe eau/butanol/éthanol venant par le conduit (11) de la colonne (20) de distillation du butanol. Le mélange est envoyé par le conduit (12), éventuellement via, par exemple, un échangeur de chaleur (13) utilisant l'eau ou l'air comme fluide refroidissant, dans un ballon de décantation (14). La phase lourde sort du ballon de décantation (14) par le conduit (15) et est entrainée, grâce à la pompe (16) par le conduit (17) vers la colonne de séparation eau-éthanol (2), à un plateau théorique proche et en aval du soutirage (10). La phase légère est entrainée par la pompe (18) à travers le conduit (19) vers la colonne (20).

La colonne de distillation (20) comprend un dispositif de reflux (ou condensation) en tête, avec par exemple un échangeur de chaleur (21), un ballon de reflux (22), une pompe de reflux (23) renvoyant le reflux à la colonne (20) par le conduit (24) et le distillat éthanol-eau liquide vers la colonne de séparation (2) par le conduit (25). La colonne (20) comprend également un dispositif de rebouillage (26) en fond. La colonne (20) comprend un soutirage latéral situé dans la zone entre l'alimentation (19) et le fond de la colonne. La coupe soutirée est envoyée par le conduit (11) vers le mélangeur, avec la solution hydroalcoolique du soutirage de la colonne (2). En fond de colonne (20), un effluent riche en butanol est extrait par le conduit (27).

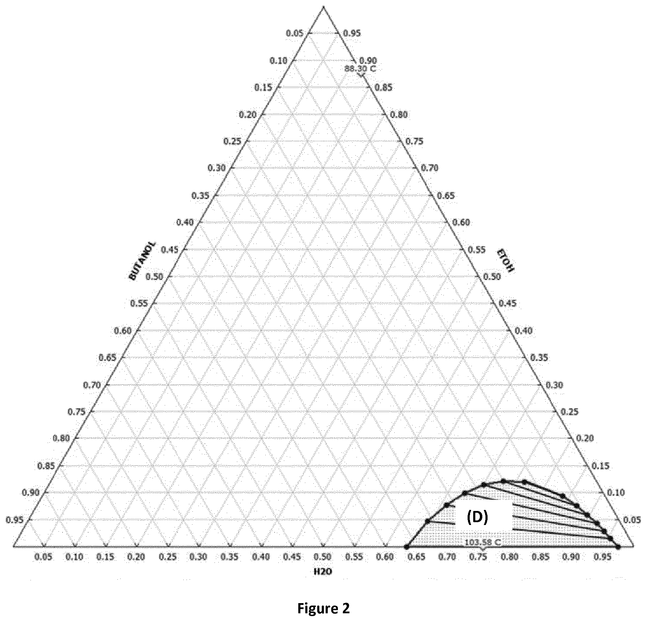

Figure 2 : diagramme ternaire eau/éthanol/butanol obtenu à une pression de 1,5 bar, avec la zone (D) de séparation de phase, appelée « loupe de démixtion ». A cette pression de 1,5 bar, la loupe de démixtion (D) du ternaire eau/éthanol/butanol se situe à des teneurs molaires comprises entre 60% et 98% en eau, entre 2% et 40% en butanol et inférieures à 14% molaire en éthanol.

### EXEMPLES

Les exemples suivants sont basés sur des simulations procédés intégrant des données thermodynamiques calées sur des points expérimentaux (en particulier données d'équilibre liquide-vapeur binaires, coefficient de partage liquide-liquide).

L'exemple 2 illustre le procédé selon l'invention, comparativement à l'Exemple 1 qui montre une simple distillation.

L'exemple 4 illustre l'application du procédé selon l'invention dans un procédé Lebedev, comparativement à l'Exemple 3 qui montre le procédé Lebedev avec une colonne de séparation eau-éthanol simple.

Conformément à l'invention, et en particulier dans les exemples ci-après, on entend par « traces » d'un composé dans un flux (comme un effluent), une teneur en ledit composé inférieure à 10 ppm mol, cette teneur étant considérée comme négligeable dans ledit flux, par exemple effluent.

### Exemple 1 (comparatif):

Une charge hydroalcoolique comprenant de l'eau, de l'éthanol et une faible teneur de butanol, est traitée par distillation.

Les caractéristiques de la charge hydroalcoolique à traiter sont les suivantes :
- 100 tonne/h (3773 Kmole/h), à une température de 90°C et une pression de 1,8 bar abs ;
- Composition molaire de la charge : 70% d'eau, 29,75% d'éthanol et 0,25% de butanol (correspondant à 699 kg/h de butanol).

La charge hydroalcoolique alimente une colonne de distillation comprenant 22 plateaux théoriques, l'alimentation étant placée au plateau théorique 15 (en partant de la tête). La colonne comprend un système de reflux. Elle a une pression de 1,1 bar abs après le condenseur de tête et 1,6 bar en fond et un taux de reflux molaire de 0,65 par rapport au distillat.

Pour cette simulation, la spécification d'éthanol en fond de colonne est fixée à 0,1 ppm molaire.

Sont extraits en tête de colonne un effluent (60.87 tonnes/h) comprenant 70,11 % mol d'éthanol, 29,30% mol d'eau et 0,59% mol de butanol et en fond de colonne de l'eau quasi pure (39.13 tonnes/h), comprenant 0,1 ppm molaire d'éthanol. Tout le butanol (699 kg/h) se retrouve dans l'effluent extrait en tête, du fait de la formation de l'azéotrope butanol-eau plus léger que l'eau (Température d'ébullition de l'azéotrope butanol-eau = 92,4°C).

### Exemple 2 (conforme à l'invention) :

La même charge hydroalcoolique que celle traitée selon l'Exemple 1 est traitée par le procédé selon l'invention (cf. Figure 1).

Les caractéristiques de la charge hydroalcoolique à traiter sont les suivantes :
- 100 tonne/h (3773 Kmole/h), à une température de 90°C et une pression de 1,8 bar abs ;
- Composition molaire de la charge : 70% d'eau, 29,75% d'éthanol et 0,25% de butanol (correspondant à 699 kg/h de butanol).

La colonne de séparation (2) comprend 33 plateaux théoriques et a un taux de reflux de 0,65. Elle est alimentée en la charge hydroalcoolique au plateau théorique 15. En fond de colonne, sont extraits 39,85 tonne/h d'eau comprenant 0,1 ppm mole d'éthanol. En tête de colonne, est extrait 59,75 tonne/h d'un effluent riche en éthanol comprenant 72,14% mol/mol d'éthanol, 27,6% mol/mol d'eau et 0,26% mol/mol de butanol. 100 Kmole/h (2,6 tonne/h) sont soutirés au niveau du plateau 26. Le liquide soutiré contient 79% mol/mol d'eau, 7,67% mol/mol de butanol et 13,33% mol/mol d'éthanol.

Le liquide soutiré de la colonne (2) est mélangé avec une coupe soutirée (11) sur la colonne (20) (soutirée entre l'alimentation et le fond) et ayant un débit de 186 Kmole/h (6,8 tonne/h) et une composition molaire de 67,06% d'eau, 32,82% de butanol et 0,13% d'éthanol. Le mélange est ensuite refroidi de 102°C jusqu'à 92°C dans un échangeur de chaleur (13).

Le mélange décante dans le ballon (14) à une pression de 1,05 bar absolu. Le flux (15) de la phase lourde de retour à la colonne (2), au plateau théorique 27, a un débit de 78 Kmole/h (1,6 tonne/h) et une composition molaire de 95,41% d'eau, 1,83% d'éthanol et 2,76% de butanol. La phase légère comprenant 62,12% mol/mol d'eau, 32,03% mol/mol de butanol et 5,85% mol/mol d'éthanol est envoyée, à un débit de 208,7 Kmole/h (7,85 tonne/h), vers la colonne (20).

Le taux de reflux molaire de la colonne (20) par rapport à sa charge est de 1,2.

En tête de colonne (20), 16,39 Kmole/h (0,64 tonne/h) sont renvoyés par le conduit (25) sur le plateau théorique 3 de la colonne (2) : la composition molaire du distillat extrait en tête de la colonne (20) est de 72,92% d'éthanol, 25,55% d'eau et 0,53% de butanol.

En fond de colonne (20) est extrait un butanol de pureté 99,99% mol/mol.

Ainsi, le procédé selon l'invention permet de récupérer 400 kg/h (5,4 Kmole/h) de butanol en fond de colonne (20) par rapport aux 699 kg/h de butanol contenus dans la charge alimentant la colonne (2).

Globalement, par rapport à une simple distillation comme illustrée dans l'Exemple 1, le procédé permet de récupérer un effluent éthanol plus concentré en éthanol (72,14% molaire au lieu de 70,11% dans l'Exemple 1), avec deux fois moins de butanol (0,26% molaire de butanol au lieu de 0,59% molaire de butanol dans l'effluent éthanol illustré dans l'Exemple 1). Le procédé selon l'invention permet également de récupérer, ici, plus de la moitié du butanol entrant dans le procédé sous forme de butanol quasi pur, et donc valorisable

### Exemple 3 (comparatif):

Cet exemple montre la simulation du procédé Lebedev décrit dans le document FR 3 026 100 dans lequel l'effluent eau-éthanol est traité par distillation simple dans une colonne de séparation.

L'unité de production est alimentée par 48670 kg/h de charge comprenant 93,3% poids d'éthanol et 6,7% poids d'eau.

Selon ce procédé Lebedev, 18850 kg/h de produit comprenant 99,84% de butadiène sont produits, en consommant au total :
- 201,3 t/h de vapeur (117.4 MW)
- 86,81 MW de chauffage par four
- 8377 kW d'électricité
- 21 t/h d'eau procédé
- 25120 m3/h d'eau de refroidissement.

Plus particulièrement, la colonne de séparation eau-éthanol du procédé Lebedev simulé comprend 30 plateaux théoriques et a un taux de reflux molaire de 1. Elle est alimentée au plateau théorique 15 par l'effluent eau-éthanol, issu de l'unité de production/séparation du butadiène du procédé global, à un débit de 167,6 tonne/h. Cet effluent eau-éthanol en entrée de la colonne de séparation a la composition molaire suivante :
- 25,39% mol d'éthanol ;
- 73,74 % mol d'eau ;
- 0,23% mol de butanol (correspondant à 1,1 tonne/h de butanol) ;
- 0,51% mol d'acide acétique ;
- 0,13% mol d'autres impuretés.

En fond de cette colonne de séparation, l'effluent extrait a la composition molaire suivante : 99,09% d'eau, 0,86% d'acide acétique et 0,05 % d'impuretés autres (traces d'éthanol et butanol).

En tête de cette colonne de séparation, 96,38 tonne/h sont extraits, avec la composition molaire suivante :
- 62% mol d'éthanol ;
- 37,2 % mol d'eau ;
- 0,55% mol de butanol (correspondant à 1,1 tonne/h de butanol) ;
- 0% mol d'acide acétique ;
- 0,25% mol d'autres impuretés.

Le rebouilleur de la colonne de séparation eau-éthanol consomme 58,4 MWh/h, soit par exemple 100 tonne/h de vapeur basse pression, et le refroidissement de la colonne nécessite 59,6 MWh/h, soit par exemple une circulation de 6921 tonnes/h d'eau de refroidissement.

Ainsi, tout le butanol entrant dans la colonne de séparation est extrait en tête de colonne avec l'effluent éthanol destiné à être recyclé vers l'unité de production du butadiène, entrainant des problèmes d'accumulation du butanol au niveau des réacteurs et une perte d'activité et de sélectivité des catalyseurs de conversion en butadiène.

Il apparait également que la consommation de vapeur de cette colonne de séparation représente environ la moitié de la consommation totale du procédé global (100 tonnes/h de vapeur par rapport à 201 au total), sans que le butanol soit séparé.

### Exemple 4 (selon l'invention):

Cet exemple simule le procédé Lebedev comme dans l'Exemple précédent, mais dans lequel a été inséré le procédé de traitement (cf. Figure 1) selon l'invention à la place de la simple colonne de séparation.

La même charge que celle dans l'Exemple 3, comprenant 93,3% poids d'éthanol et 6,7% poids d'eau, alimente l'unité de production à un débit de 48670 kg/h. L'unité de traitement de l'effluent eau-éthanol issu de l'unité de conversion comprend la section de séparation (étape a) du procédé selon l'invention), les sections de mélanges et de décantation (étape b) du procédé selon l'invention) et la section de séparation du butanol (étape c).

La colonne de séparation eau-éthanol (2) comprend 30 plateaux théoriques et a un taux de reflux molaire de 0,779 Elle est alimentée par l'effluent eau-éthanol issu de l'unité de conversion au plateau théorique 15. Dans ce cas-là, l'effluent eau-éthanol à l'entrée de la colonne de séparation (2) a un débit 158,5 tonne/h et la composition molaire suivante :
- Ethanol : 26,94% mol ;
- Eau : 72,27 % mol ;
- Butanol : 0,17% mol (correspondant à 787 kg/h de butanol) ;
- Acide acétique : 0,53% mol ;
- Autres impuretés : 0,09% mol.

Un effluent riche en eau est extrait en fond de colonne (2). Il a la composition molaire suivante :
- Ethanol : traces ;
- Eau : 99,15 % mol ;
- Butanol : traces ;
- Acide acétique : 0,85% mol ;
- Autres impuretés : traces.

Le produit extrait en tête de colonne (2) est recyclé vers le réacteur de conversion de l'éthanol en butadiène à un débit de 88,69 tonne/h. Il a la composition molaire suivante :
- Ethanol : 71,02% mol ;
- Eau : 28,40 % mol ;
- Butanol : 0,33% mol (correspondant à 574 kg/h de butanol) ;
- Acide acétique : 0,0% mol ;
- Autres impuretés : 0,25% mol.

La colonne de séparation comprend aussi un soutirage latéral au plateau théorique 23, d'un liquide à un débit de 3772 kg/h (soit 161,5 kmol/h) et comprenant 4,6% mol de butanol (soit un débit de butanol de 550 kg/h), 8,99% mol d'éthanol, 85,84% mol d'eau, 0,57% mol d'acide acétique et des traces d'impuretés.

Le liquide soutiré sur la colonne de séparation (2) est mélangé à la coupe soutirée sur la colonne (20). Le mélange est ensuite refroidi à 92,5°C dans un échangeur de chaleur (13) puis envoyé dans le ballon de décantation (14) dont la pression est 1,05 bars.

La phase lourde soutirée du ballon décanteur a un débit de 2776 kg/h et une composition molaire de 95,35% d'eau, 1,16% d'éthanol, 2,88% de butanol, et 0,6% d'acide acétique (traces d'impuretés). Ce flux est envoyé au plateau 24 de la colonne 2.

La phase légère soutirée du ballon décanteur a un débit de 20040 kg/h et une composition molaire de 62,17% d'eau, 3,71% d'éthanol, 33,39% de butanol, et 0,74% d'acide acétique (traces d'impuretés). Ce flux est la charge de la colonne (20).

La colonne (20) comporte 22 plateaux théoriques et est alimentée au plateau 9. Le taux de reflux molaire de cette colonne (20) est de 11,76.

Un soutirage latéral est effectué sur la colonne (20) au plateau théorique 17. La coupe soutirée sur la colonne (20) est envoyée vers la section de mélange à un débit de 19,05 tonne/h (soit 502 kmol/h). Sa composition molaire est : 63,63% d'eau, 1,31% d'éthanol, 34,31% de butanol et 0,76% d'acide acétique (traces d'impuretés).

Le distillat extrait en tête de colonne (20) est renvoyé vers la colonne (2) de séparation eau-éthanol à un débit de 780 kg/h. Sa composition molaire est : 60,84% d'éthanol, 36,34% d'eau, 2,82% de butanol et des traces d'acide acétique (traces d'impuretés autres).

L'effluent en fond de colonne (20) est extrait à un débit de 218 kg/h et a la composition molaire suivante :
- Ethanol : traces
- Eau : traces
- Butanol : 97,15% mol
- Acide acétique : 2,85% mol
- Autres impuretés : traces.

Les consommations d'utilités sont 43,88 MW pour le rebouillage de la colonne (2) et de 3,28 MW pour le rebouillage de la colonne (20) de séparation du butanol, soit un total de 47,16 MW.

Le tableau 1 ci-dessous résume les résultats de l'unité de traitement de l'effluent eau-éthanol du procédé Lebedev utilisant le procédé de traitement selon l'invention et les compare à ceux de l'unité de traitement comprenant une colonne de distillation simple (Exemple 3 comparatif).

**Tableau 1 : Résultats des unités de traitement de l'effluent eau-éthanol utilisant le procédé de selon l'invention ou utilisant une colonne de distillation simple (Exemple 3 comparatif)**

| | | Avec le procédé selon l'invention | Avec une distillation simple |
|---|---|---|---|
| Charge de l'unité | Débit total (tonne/h) | 158,5 | 167,6 |
| | Débit BuOH (tonne/h) | 0,787 | 1,1 |
| Effluent extrait en tête de colonne eau-éthanol et recyclé | Débit (tonne/h) | 88,69 | 96,38 |
| | Teneur en EtOH (%mol) | 71,02 | 62 |
| | Teneur en eau (%mol) | 28,40 | 37,2 |
| | Teneur en butanol (%mol) | 0,33 | 0,55 |
| | Débit de butanol recyclé (tonne/h) | 0,574 | 1,1 |
| Effluent riche en butanol extrait | | 218 kg/h à 97,15% mol de BuOH | NA |
| Consommation énergétique pour le rebouillage (MW) | | 47,16 | 58,4 |

Lorsque le procédé selon l'invention est utilisé dans l'unité de traitement de l'effluent eau-éthanol du procédé Lebedev, il apparait que :
- la quantité de charge hydroalcoolique (158,5 tonne/h) alimentant la colonne (2) de séparation eau-éthanol est diminuée par rapport à la quantité de charge (167,6 tonne/h) d'une simple colonne de séparation comme celle de l'Exemple 3. Ceci s'explique par une quantité d'eau recyclée avec l'éthanol dans l'unité de réaction de conversion en butadiène diminuée (28,40% mol d'eau par rapport à 37,2% mol) ;
- l'effluent riche en éthanol, récupéré en tête de colonne (2) de séparation eau-éthanol, a un débit plus faible (88, 69 tonne/h par rapport à 96,38 tonne/h) ;
- l'effluent riche en éthanol, récupéré en tête de colonne (2) contient plus d'éthanol (71,02% mol) avec un gain de plus de 10 points par rapport la composition de l'effluent éthanol dans le cas d'une simple distillation (62% mol) ;
- une partie du butanol entrant dans l'unité de traitement est extrait dans le cas du procédé selon l'invention alors que tout le butanol est recyclé vers le réacteur dans le cas d'une distillation simple. La pureté du butanol, récupéré en fond de colonne (20), est très satisfaisante et permet d'envisager une valorisation du butanol (par exemple commercialisation du butanol, éventuellement comme butanol bio-sourcé) ;
- la consommation d'utilités pour le rebouillage est diminuée de 20% dans le cas du procédé selon l'invention (47,16 MW) par rapport au cas de distillation simple (58,4 MW).

Dans le cas de l'intégration du procédé de traitement selon l'invention dans le procédé Lebedev, les consommations d'utilités totales sont pour l'ensemble du procédé Lebedev, en comparaison de celles obtenues pour le procédé décrit dans l'Exemple 3 :
- 181,4 t/h de vapeur (soit 105.8 MW), soit une diminution de 10% ;81 MW de chauffage par four, soit une diminution de 6,7% ;
- 8168 kW d'électricité, soit une diminution de 2,5% ;
- 21 t/h d'eau procédé (inchangé) ;
- 22125 m3/h d'eau de refroidissement, soit une diminution de 12%.

Il apparait clairement que des économies sur les utilités peuvent être faites dans le cas du procédé Lebedev incluant le procédé de traitement selon l'invention.

De plus, en incluant un tel procédé de traitement dans le procédé Lebedev, dans la mesure où le butanol est extrait partiellement, les performances catalytiques pourront être améliorées. Une diminution de la quantité d'impuretés lourdes résultant de la dégradation du butanol ainsi qu'une limitation des effluents qui pourraient démixer dans le procédé pourront être également observées.

Dans le cas du procédé Lebedev intégrant le procédé de traitement selon l'invention, l'effluent riche en éthanol recyclé vers l'unité réactionnelle du procédé Lebedev étant plus concentré en éthanol (moins d'eau et moins de butanol) permet un gain d'utilités dans les sections réactionnelles (gain au niveau de la vaporisation de la charge) et lors des traitements en aval des sections réactionnelles (gain au niveau des distillations et lavages), par rapport au procédé Lebedev sans le procédé de traitement selon l'invention.

## Revendications

1. Procédé de traitement d'une charge hydroalcoolique (1) comprenant de l'eau, de l'éthanol et du butanol, pour produire un effluent riche en éthanol (7), un effluent riche en eau (9) et un effluent riche en butanol (27), comprenant :
a) une étape de séparation eau-éthanol, comprenant une section de séparation eau-éthanol comprenant une colonne de distillation (2) comprenant au moins 14 plateaux théoriques et ayant un taux de reflux inférieur ou égal à 1,2, ladite section de séparation eau-éthanol comprenant :
∘ une alimentation en ladite charge hydroalcoolique (1), située au niveau ou en aval du troisième plateau théorique,
∘ le soutirage latéral d'une solution hydroalcoolique (10) comprenant du butanol, dans la zone d'accumulation du butanol de la colonne de distillation, située en aval de l'alimentation,
∘ une injection de la phase lourde (17) issue de l'étape b), située en aval et dans une zone comprise entre le soutirage latéral et le troisième plateau théorique en aval du soutirage latéral,
∘ une injection du distillat éthanol-eau (25) issu de l'étape c), située en amont de l'alimentation et dans une zone de la colonne de distillation comprise entre le plateau théorique en tête de colonne au niveau duquel revient le reflux (6) et le troisième plateau théorique,
ladite section de séparation eau-éthanol produisant en tête de colonne un effluent riche en éthanol (7) et en fond de colonne un effluent riche en eau (9);
b) une étape de démixtion comprenant
∘ une section de mélange de ladite solution hydroalcoolique (10) soutirée à l'étape a) avec la coupe eau/butanol/éthanol (11) soutiré à l'étape c) ;
∘ une section de décantation du mélange (12) précédent comprenant un ballon de décantation (14) dans lequel la pression est inférieure ou égale à 6 bars absolus, ladite section de décantation produisant une phase lourde (15, 17) comportant principalement de l'eau et une phase légère (19) ;
c) une étape de séparation du butanol, comprenant une section de distillation du butanol comprenant au moins une colonne de distillation (20) alimentée par la phase légère (19) issue de l'étape b) et comprenant un soutirage latéral, situé sur ladite colonne (20) dans une zone comprise entre l'alimentation et le fond de la colonne, d'une coupe eau/butanol/éthanol (11), ladite section de distillation du butanol produisant en fond de colonne un effluent riche en butanol (27) et en tête de colonne un distillat éthanol-eau (25).

2. Procédé selon la revendication 1, dans lequel le butanol de la charge hydroalcoolique (1) représente moins de 5% mol/mol, en particulier moins de 2% mol/mol, plus particulièrement moins de 1% mol/mol ou encore moins de 0,5% mol/mol de la charge hydroalcoolique.

3. Procédé selon la revendication 1 ou 2, dans lequel la colonne de distillation (2) de la section de séparation de l'étape a) comprend entre 20 et 35 plateaux théoriques.

4. Procédé selon l'une des revendications précédentes, dans lequel la colonne de distillation (2) de la section de séparation de l'étape a) a un taux de reflux molaire compris entre 0,5 et 1,15, de préférence entre 0,65 et 1,15.

5. Procédé selon l'une des revendications précédentes, dans lequel la colonne de distillation (2) de la section de séparation de l'étape a) est alimentée en ladite charge hydroalcoolique (1) dans une zone comprise entre le tiers et les deux tiers de la hauteur totale de ladite colonne.

6. Procédé selon l'une des revendications précédentes, dans lequel le soutirage latéral de l'étape a) se situe dans une zone comprise entre le tiers et les deux tiers de la hauteur de la zone de fond de la colonne, ladite zone de fond étant la zone située entre l'alimentation et le fond de la colonne.

7. Procédé selon l'une des revendications précédentes, dans lequel la solution hydroalcoolique (10) soutirée à l'étape a) représente entre 15 et 40 % mol/mol du mélange (12) de la section de mélange de l'étape b).

8. Procédé selon l'une des revendications précédentes, dans lequel la pression dans le ballon de décantation (14) est comprise entre 1 et 2 bars absolus.

9. Procédé selon l'une des revendications précédentes, dans lequel le mélange (12) issu de la section de mélange de l'étape b) est refroidi (13) à une température comprise entre 80 et 95°C, avant d'être envoyé dans le ballon de décantation (14).

10. Procédé Lebedev comprenant une étape de traitement utilisant le procédé selon l'une quelconques des revendications précédentes, dans lequel la charge hydroalcoolique est l'effluent eau-éthanol issu de l'unité réactionnelle dudit procédé Lebedev et l'effluent riche en éthanol produit à l'étape a) est recyclé vers l'unité réactionnelle.

## Patentansprüche

1. Verfahren zur Behandlung einer wässrig-alkoholischen Beschickung (1), die Wasser, Ethanol und Butanol umfasst, zum Herstellen eines ethanolreichen Abstroms (7), eines wasserreichen Abstroms (9) und eines butanolreichen Abstroms (27), umfassend:
a) einen Wasser-Ethanol-Trennungsschritt, umfassend einen Wasser-Ethanol-Trennungsabschnitt, der eine Destillationskolonne (2) umfasst, die mindestens 14 theoretische Böden und ein Rückflussverhältnis von weniger als oder gleich 1,2 umfasst, wobei der Wasser-Ethanol-Trennungsabschnitt Folgendes umfasst:
eine Zufuhr der wässrig-alkoholischen Beschickung (1), die sich auf Höhe oder stromabwärts des dritten theoretischen Bodens befindet,
den Seitenabzug einer Butanol umfassenden wässrig-alkoholischen Lösung (10) in der Butanol-Akkumulationszone der Destillationskolonne, die sich stromabwärts der Zufuhr befindet,
eine Einspritzung der aus Schritt b) stammenden schweren Phase (17), die sich stromabwärts und in einer Zone zwischen dem Seitenabzug und dem dritten theoretischen Boden stromabwärts des Seitenabzugs befindet,
eine Einspritzung des aus Schritt c) stammenden Ethanol-Wasser-Destillats (25), die sich stromaufwärts der Zufuhr und in einer Zone der Destillationskolonne zwischen dem theoretischen Boden am Kopf der Kolonne, in dessen Höhe der Rückfluss (6) zurückfließt, und dem dritten theoretischen Boden befindet,
wobei der Wasser-Ethanol-Trennungsabschnitt am Kopf der Kolonne einen ethanolreichen Abstrom (7) und im Kolonnensumpf einen wasserreichen Abstrom (9) produziert;
b) einen Entmischungsschritt, umfassend
einen Abschnitt des Mischens der in Schritt a) abgezogenen wässrig-alkoholischen Lösung (10) mit der in Schritt c) abgezogenen Wasser/Butanol/Ethanol-Fraktion (11) ;
einen Abschnitt des Dekantierens des vorherigen Gemischs (12), der einen Dekantierballon (14) umfasst, in dem der Druck kleiner als oder gleich 6 bar absolut ist, wobei der Dekantierabschnitt eine schwere Phase (15, 17), die hauptsächlich Wasser enthält, und eine leichte Phase (19) produziert;
c) einen Butanol-Abtrennungsschritt, umfassend einen Butanol-Destillationsabschnitt, der mindestens eine Destillationskolonne (20) umfasst, die mit der aus Schritt b) stammenden leichten Phase (19) beschickt wird, und einen Seitenabzug einer Wasser/Butanol/Ethanol-Fraktion (11) umfasst, der sich an der Kolonne (20) in einer Zone zwischen der Zufuhr und dem Sumpf der Kolonne befindet, wobei der Butanol-Destillationsabschnitt im Kolonnensumpf einen butanolreichen Abstrom (27) und am Kopf der Kolonne ein Ethanol-Wasser-Destillat (25) produziert.

2. Verfahren nach Anspruch 1, wobei das Butanol der wässrig-alkoholischen Beschickung (1) weniger als 5 % Mol/Mol, insbesondere weniger als 2 % Mol/Mol, genauer gesagt weniger als 1 % Mol/Mol oder sogar weniger als 0,5 % Mol/Mol der wässrig-alkoholischen Beschickung ausmacht.

3. Verfahren nach Anspruch 1 oder 2, wobei die Destillationskolonne (2) des Trennungsabschnitts von Schritt a) zwischen 20 und 35 theoretische Böden umfasst.

4. Verfahren nach einem der vorhergehenden Ansprüche, wobei die Destillationskolonne (2) des Trennungsabschnitts von Schritt a) ein molares Rückflussverhältnis zwischen 0,5 und 1,15, vorzugsweise zwischen 0,65 und 1,15 aufweist.

5. Verfahren nach einem der vorhergehenden Ansprüche, wobei die Destillationskolonne (2) des Trennungsabschnitts von Schritt a) mit der wässrig-alkoholischen Beschickung (1) in einer Zone zwischen einem Drittel und zwei Dritteln der Gesamthöhe der Kolonne beschickt wird.

6. Verfahren nach einem der vorhergehenden Ansprüche, wobei sich der Seitenabzug von Schritt a) in einer Zone zwischen einem Drittel und zwei Dritteln der Höhe der Sumpfzone der Kolonne befindet, wobei die Sumpfzone die Zone ist, die sich zwischen der Zufuhr und dem Sumpf der Kolonne befindet.

7. Verfahren nach einem der vorhergehenden Ansprüche, wobei die in Schritt a) abgezogene wässrig-alkoholische Lösung (10) zwischen 15 und 40 % Mol/Mol des Gemischs (12) des Mischabschnitts von Schritt b) ausmacht.

8. Verfahren nach einem der vorhergehenden Ansprüche, wobei der Druck im Dekantierballon (14) zwischen 1 und 2 bar absolut beträgt.

9. Verfahren nach einem der vorhergehenden Ansprüche, wobei das aus dem Mischabschnitt von Schritt b) stammende Gemisch (12) auf eine Temperatur zwischen 80 und 95 °C abgekühlt wird (13), bevor es in den Dekantierballon (14) geleitet wird.

10. Lebedev-Verfahren, umfassend einen Behandlungsschritt unter Verwendung des Verfahrens nach einem der vorhergehenden Ansprüche, wobei die wässrig-alkoholische Beschickung der aus der Reaktionseinheit des Lebedev-Verfahrens stammende Wasser-Ethanol-Abstrom ist und der in Schritt a) produzierte ethanolreiche Abstrom zur Reaktionseinheit zurückgeführt wird.

## Claims

1. Process for treating an aqueous-alcoholic feedstock (1) comprising water, ethanol and butanol in order to produce an ethanol-rich effluent (7), a water-rich effluent (9) and a butanol-rich effluent (27), comprising:
a) a water-ethanol separation step, comprising a water-ethanol separation section comprising a distillation column (2) comprising at least 14 theoretical plates and having a reflux ratio of less than or equal to 1.2, said water-ethanol separation section comprising:
o a feed of said aqueous-alcoholic feedstock (1), located at the level of or downstream of the third theoretical plate,
o the side withdrawal of an aqueous-alcoholic solution (10) comprising butanol in the butanol accumulation zone of the distillation column located downstream of the feed,
o an injection of the heavy phase (17) resulting from step b), located downstream and in a zone between the side withdrawal and the third theoretical plate downstream of the side withdrawal,
o an injection of the ethanol-water distillate (25) resulting from step c), located upstream of the feed and in a zone of the distillation column between the theoretical plate at the top of the column at which the reflux (6) returns and the third theoretical plate,
said water-ethanol separation section producing, at the top of the column, an ethanol-rich effluent (7) and, at the bottom of the column, a water-rich effluent (9);
b) a demixing step comprising
o a section for mixing said aqueous-alcoholic solution (10) withdrawn in step a) with the water/butanol/ethanol fraction (11) withdrawn in step c);
o a section for decanting the previous mixture (12), comprising a decantation tank (14) in which the pressure is less than or equal to 6 bar absolute, said decanting section producing a heavy phase (15, 17) mainly containing water and a light phase (19);
c) a butanol separation step, comprising a butanol distillation section comprising at least one distillation column (20) fed with the light phase (19) resulting from step b) and comprising a side withdrawal, located on said column (20) in a zone between the feed and the bottom of the column, of a water/butanol/ethanol fraction (11), said butanol distillation section producing, at the bottom of the column, a butanol-rich effluent (27) and, at the top of the column, an ethanol-water distillate (25).

2. Process according to Claim 1, wherein the butanol of the aqueous-alcoholic feedstock (1) represents less than 5% mol/mol, in particular less than 2% mol/mol, more particularly less than 1% mol/mol or even less than 0.5% mol/mol, of the aqueous-alcoholic feedstock.

3. Process according to Claim 1 or 2, wherein the distillation column (2) of the separation section of step a) comprises between 20 and 35 theoretical plates.

4. Process according to one of the preceding claims, wherein the distillation column (2) of the separation section of step a) has a molar reflux ratio of between 0.5 and 1.15, preferably between 0.65 and 1.15.

5. Process according to one of the preceding claims, wherein the distillation column (2) of the separation section of step a) is fed with said aqueous-alcoholic feedstock (1) in a zone between one-third and two-thirds of the total height of said column.

6. Process according to one of the preceding claims, wherein the side withdrawal of step a) is located in a zone between one-third and two-thirds of the height of the bottom zone of the column, said bottom zone being the zone located between the feed and the bottom of the column.

7. Process according to one of the preceding claims, wherein the aqueous-alcoholic solution (10) withdrawn in step a) represents between 15 and 40% mol/mol of the mixture (12) of the mixing section of step b).

8. Process according to one of the preceding claims, wherein the pressure in the decantation tank (14) is between 1 and 2 bar absolute.

9. Process according to one of the preceding claims, wherein the mixture (12) obtained from the mixing section of step b) is cooled (13) to a temperature between 80 and 95°C, before being sent to the decantation tank (14).

10. Lebedev process comprising a treatment step utilizing the process according to any one of the preceding claims, wherein the aqueous-alcoholic feedstock is the water-ethanol effluent obtained from the reaction unit of said Lebedev process and the ethanol-rich effluent produced in step a) is recycled to the reaction unit.
